# EUROPEAN PATENT SPECIFICATION

(11) **EP 1 301 784 B1**
(45) Date of publication and mention of the grant of the patent: **05.10.2005**
(21) Application number: 01949722.1
(22) Date of filing: 17.07.2001
(51) Int. Cl.: G01N 33/487

(54) **DEVICE FOR MEASURING ANALYTE CONCENTRATION IN A FLUID**
VORRICHTUNG ZUR MESSUNG DER KONZENTRATION EINES ANALYTS IN EINER FLÜSSIGKEIT
DISPOSITIF D'ESSAI

(30) Priority: 20.07.2000 GB 0017737
(43) Date of publication of application: 16.04.2003
(73) Proprietor: HYPOGUARD LIMITED, Suffolk IP12 1PE (GB)
(72) Inventor: WOODHEAD, Andrew James, London WC1H 9NX (GB); MAY, Stuart Richard, Surrey KT8 1QZ (GB); BLACK, Murdo M., Ipswich IP12 4SH (GB)
(74) Representative: Gemmell, Peter Alan
(86) International application number: PCT/GB2001/003195
(87) International publication number: WO 2002/008753

(56) References cited:
- EP-A- 0 732 590
- WO-A-94/10558
- DE-A- 19 639 226

## Description

### BACKGROUND OF THE INVENTION

### 1. Field of the Invention

The present invention relates to a test device for measuring the concentration of an analyte in a fluid sample, notably to a test device for analysing blood glucose or other analytes in bodily fluids.

### 2. Description of the Prior Art

Diabetics regularly need to test samples of their blood to determine the level of blood glucose. The results of such tests may be used to determine levels of medication needed to treat the diabetes at the time. In one known type of system, disposable sensors are used to test the blood. The sensors typically take the form of test strips which are provided with a reagent material that will react with blood glucose to produce an electrical signal. Conductive tracks on the test strip relay the electrical signal to a meter which displays the result. After a sample of blood has been applied to the test strip and the measurement has been taken, the test strip is disposed of. In order to couple the conductive tracks on a test strip with the meter, the test strip needs to be inserted into a sensor holder prior to the start of testing. The sensor holder has corresponding electrodes which are brought into electrical contact with the conductive tracks of the test strip. Test devices are known in which a plurality of test strip are provided on a cartridge disc. Each strip is housed in its own sensor slot, and means are provided to eject a test strip from its slot when required, and to automatically locate it in a sensor holder. Examples of test devices with test strip dispensers are described in US Patent No. 5,660,791, European Patent Application No. 0 732 590, and European Patent Application No. 0 738 666.

A problem with test strips is that they have only a limited shelf life, and exposure of test strips to the atmosphere reduces the shelf life further. Test strips open to the atmosphere will typically have a shelf life of about two to three months, whereas test strips which are sealed from the atmosphere will have a shelf life of about six to 12 months.

Article 54(3) document EP 1 271 136 A1 discloses a test device in which a moving cutter pierces the protective seal prior to the pusher pushing a test strip from the cartridge.

It has been proposed in WO 94/10558 to provide a stack of disposable test elements in a cylindrical housing, the stack being urged towards a test station to form a liquidproof seal. In DE 196 39 226 A1 it is proposed to provide a test device with a cartridge that may have a plurality of chambers containing test strips, each of which chambers may be individually sealed to preserve the shelf life of the strips therein. A user removes the seal for each chamber when required, and a timing circuit may be activated either by the user or when the cartridge is pushed into the device. After a set time period has elapsed, an alarm or other indication reminds the user that the time period for using the strips has elapsed.

It is an object of the present invention to provide an improved test device.

### SUMMARY OF THE INVENTION

According to the present invention there is provided a test device for testing of analyte concentration in a fluid to be applied thereto, as specified in claim 1. Preferred features are specified in the dependent claims.

The device may be disposable and may be pre-loaded with all the test members. At the point of final assembly, test member and meter calibration constants, expiry date information and units of measurement may be downloaded into the meter by suitable means, for example via a bidirectional RS232 bus. This has the advantage that each meter may be uniquely matched to the specific batch of test members used within the device. The meter and associated test members can be quality controlled as a single unit and by removing the need for a user to input calibration data, as in a conventional meter, user error of this function may be eliminated.

The software may also prevent the device from being used outside the overall product expiry date and may be programmed to prevent test members from an opened magazine from being used beyond its specific expiry period.

Because the seal on a magazine is automatically broken only when the first test member is to be used, the shelf life of each stack is maximised, and the user is not required to take any action other than to operate the device.

The test members will typically be elongate test strips, and the invention will be described herein with reference to such test strips. However it will be understood that the test members may be of any desired shape and profile.

According to the invention, a plurality of magazines are provided in a movable cartridge. Each magazine is sealed until a test strip therein is required for use, whereupon the cartridge moves to bring the magazine into a position for use, and the seal is broken. This process is preferably automated and requires no user input. Any number of magazines may be employed, each containing any desired number of test strips. For example, each magazine may have 20 test strips and there may be 5 magazines, so that the device is usable for 100 readings before being discarded.

The actuation member may be mechanically linked to the pusher, directly or indirectly, or it may be linked electronically, for example by actuating an electric motor which drives the pusher. In a preferred embodiment the actuation member comprises a plunger which the user presses. The plunger may act on the pusher via another member, notably a sliding member which has a cam surface. It will be appreciated that many other suitable arrangements may also be employed.

The seal may be broken on one side by a blade past which the magazine is moved, to permit a test strip to be pushed out. The seal may be broken at the other side and along the top by one or more cutting surfaces at the leading edge of the pusher, so that the pusher initially both cuts the seal and pushes the top test strip to the engagement location. However, it would also be possible for the pusher to cause the test strip to break through the seal without the need for a separate blade. To facilitate this, the region of the seal through which the test strip will pass may be provided with a frangible line of weakness.

In a preferred embodiment, each test strip comprises a base member having a working area to which the fluid is to be applied, containing the reagent means, and a non-working area adjacent to the working area, wherein the total thickness of the test member in at least a portion of the non-working area is at least as great as the total thickness of the test member in the working area.

By making the non-working area at least as thick as the working area, scuffing or abrasion of the working area in a stack can be reduced. Moreover, if a compressive load is applied to a stack of the test members, this may be spread out over a greater area, thereby reducing the possibility of compressive damage to the working area.

In a preferred embodiment, at least a part of the non-working area is of greater total thickness than the thickness of the working area. This further reduces the likelihood of damage to the working area by scuffing or abrasion when in a stack. The difference in thickness is preferably from 1 to 20 µm, notably from 5 to 10 µm.

To build up the working area, a plurality of layers are sequentially applied to the base layer, for example by screen printing, typically with curing or drying steps between the application steps. The layers which are printed typically comprise electrode patterns, a reagent layer, and a mesh layer (for spreading out an applied fluid). As a result of the application of these layers, the working area of a conventional electrochemical test strip is typically about 100 µm thicker than the non-working area, which contains the electrode tracks and, typically, a dielectric layer. A stack of 100 test strips will therefore be about 10 mm thicker in the working area than in the non-working area. In a test strip in accordance with the present invention, at least a part of the non-working area may be made thicker by any suitable means. Suitable means include, for example: a printed relief ink; an applied pad or tape; embossing of the base layer or an intermediate layer; or an extension of the mesh layer from the working area.

Further objects and advantages of the invention will be apparent as the description proceeds.

### BRIEF DESCRIPTION OF THE DRAWINGS

The invention will now be further described by way of example with reference to the following drawings, in which:
Figure 1 illustrates user actions in operating a test device in accordance with the present invention;
Figure 2 shows the assembly of a cartridge for use in the device of Figure 1;
Figure 3 illustrates the action of the pusher in opening the seal on a magazine;
Figure 4 illustrates the action of the pusher on a test strip;
Figure 5 shows a mechanism for operating the pusher;
Figure 6 is an exploded view of part of the device of Figure 1;
Figure 7 is an exploded schematic view illustrating the cartridge advance mechanism;
Figure 8 shows steps in the advance of a cartridge;
Figure 9 shows sectional views through part of the cartridge advance mechanism at different stages;
Figure 10 illustrates an alternative embodiment in which magazines are releasably connected together; and
Figure 11 is a top plan view of a test strip in a preferred embodiment of the invention.

### DETAILED DESCRIPTION

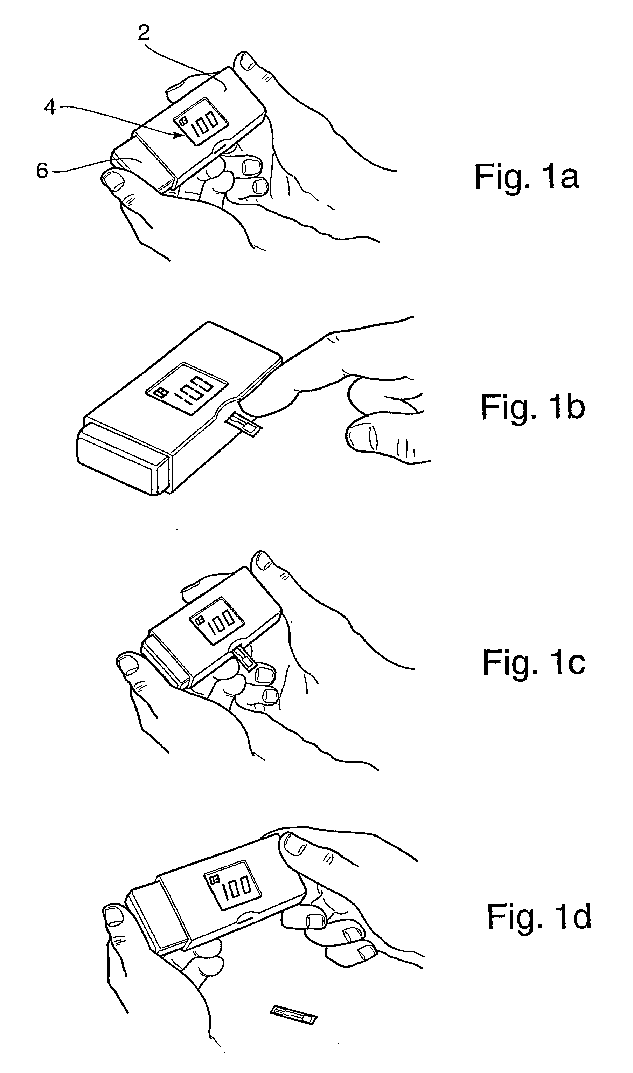

The test device shown in Figure 1 comprises a housing 2 which houses a meter with a visible display 4, in this example, an LCD. A plunger 6 is released for use by the user pressing a release button which operates a catch. Figure 1a shows the device after a user has depressed the release button with his right index finger. To take a reading of blood glucose concentration, the user partially depresses the plunger 6 to an intermediate position, causing a test strip to be presented for receiving a sample of blood (Figure 1b). After the blood has been applied to the test strip, a reading is displayed on the display 4 (Figure 1c). The user then fully depresses the plunger 6 so that it is again engaged by the catch, causing the test strip to be ejected. The device is then ready for another reading to be taken. Figure 1d illustrates the device after the test strip has been ejected and after the user has again pressed the release button to free the plunger 6. The various mechanisms involved in this process will be described below.

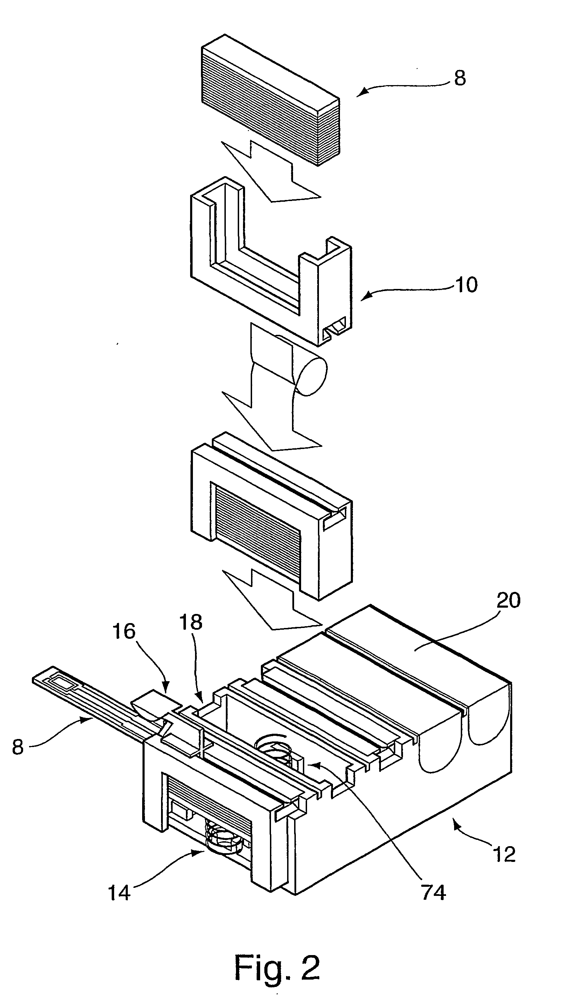

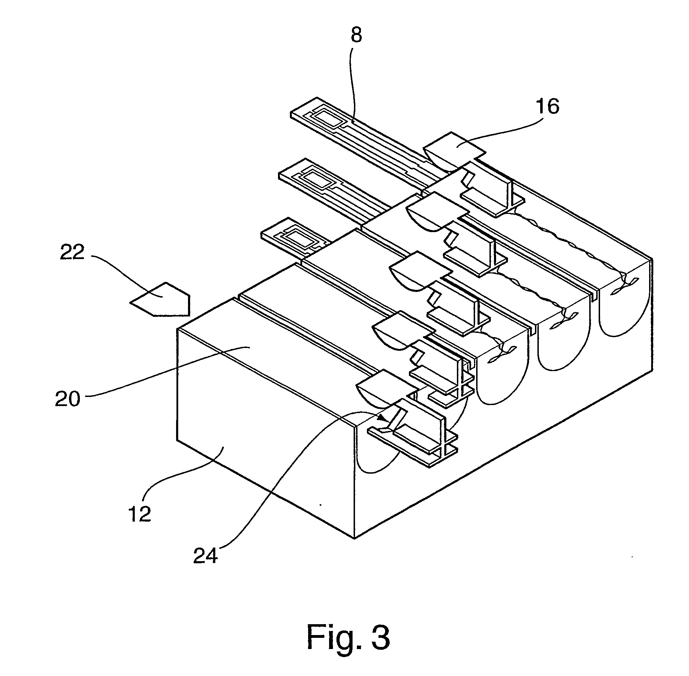

As shown in Figure 2, a stack of test strips 8 is loaded in a magazine 10, which is in turn located in cavity 74 in a cartridge 12. In this example there are five cavities 74, each of which houses spring means 14, in this example a helical spring. Each cavity has an opening 18 through which a test strip 8 will be pushed by a pusher 16. The spring urges the stack of test strips 8 upwards so that the top strip is engageable by the pusher 16. A foil seal 20 seals the cavities 74 containing the magazines 10. The slitting of the foil seal 20 will be described below, with reference to Figure 3, which shows progressive movement of the pusher 16.

Advancement of the cartridge 12 moves it past a blade 22 which is mounted in the housing 2 of the test device. The blade 22 makes a slit in the foil seal 20 at one end, through which a test strip 8 is to be pushed. The pusher 16 is moved from one end of the cavity 74 to the other, as illustrated by positions (1) through (5) of Figure 3. As the pusher 16 moves, cutting surfaces 24 on its leading edge cut the foil seal 20 and push the uppermost test strip 8 out through the opening 18 to the testing position (4) (corresponding to that shown in Figure 1b), where a test reading can be taken. Fully depressing the plunger 6 moves the pusher 16 further towards the opening 18 and ejects the test strip 8. In subsequent drawings, the seal 20 is omitted for clarity.

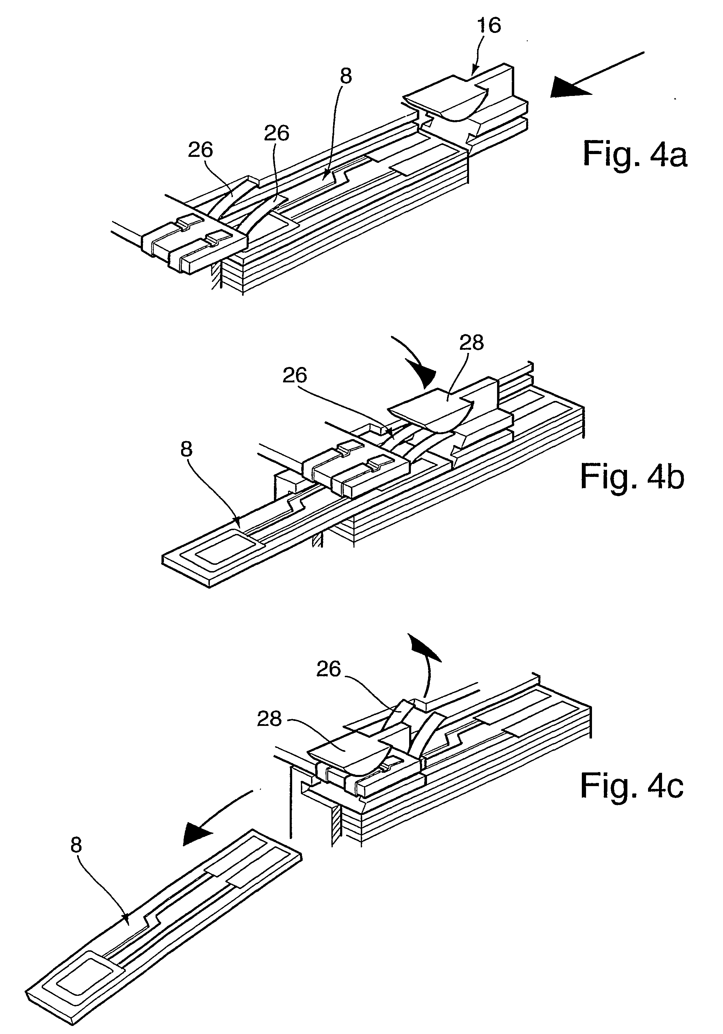

Referring now to Figure 4, a mechanism is illustrated whereby movement of the pusher 16 from the position in Figure 4a initially pushes the test strip 8 out of the housing and then the head 28 of the pusher 16 bears down on the electrodes 26 of the meter so as to bring them into engagement with electrode tracks on the test strip 8 (Figure 4b). After a reading has been taken, further advance of the pusher 16 (Figure 4c) takes the head 28 beyond the electrodes 26, which disengage from the test strip 8 and permit the pusher 16 to eject the test strip.

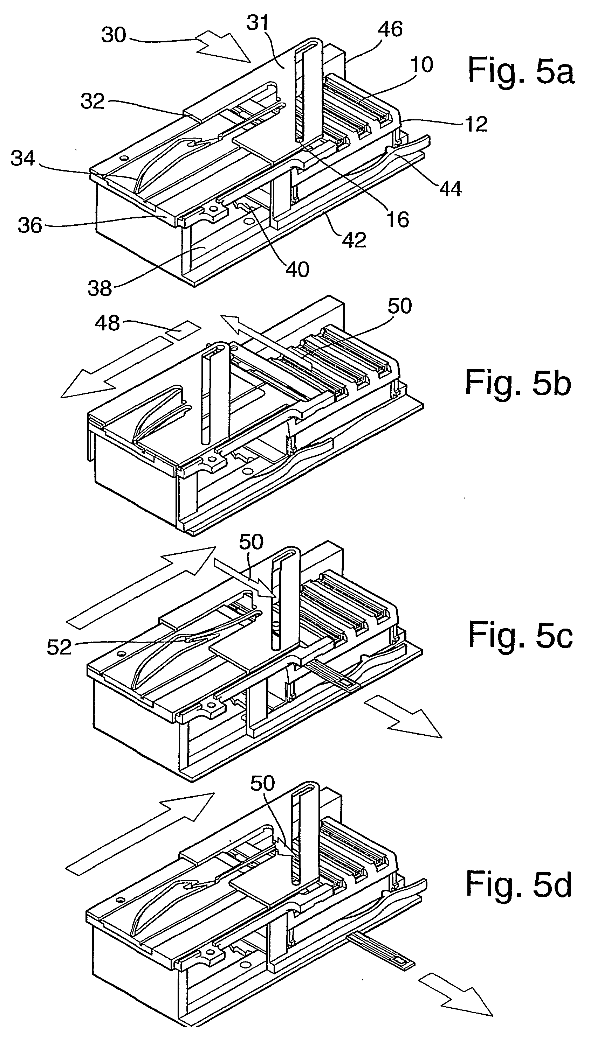

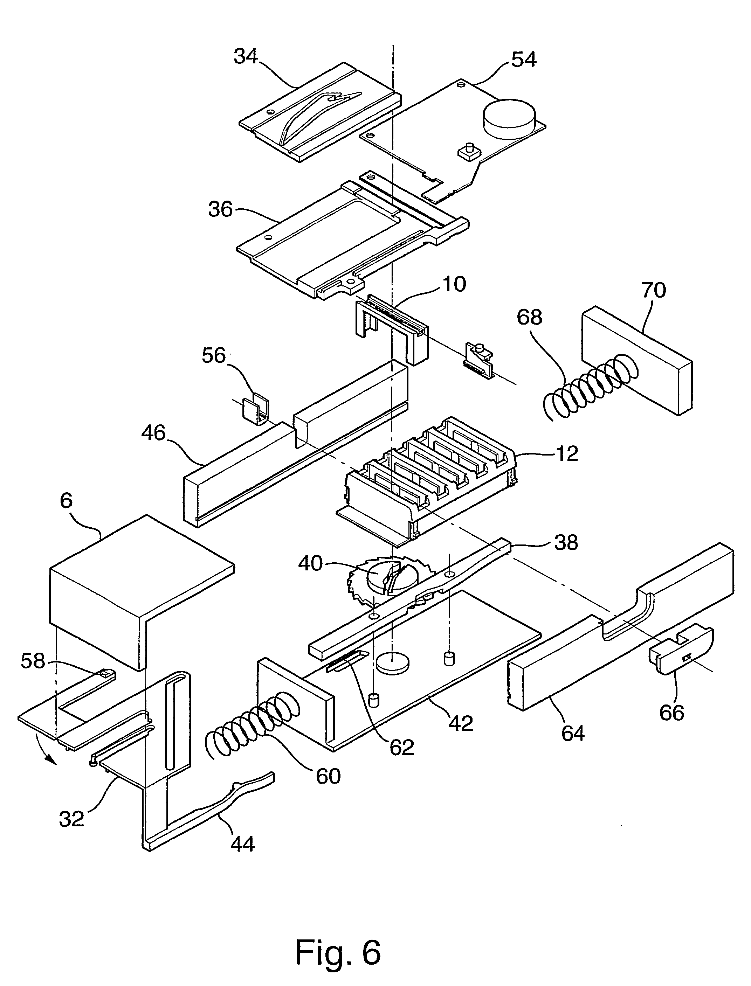

The mechanism for advancing the pusher 16 is illustrated in Figure 5. The assembly shown in Figure 5 comprises a slider 32 which is operatively connected to the plunger 6. The slider 32 is slidably mounted on a chassis top 36 which receives a groove plate 34. The groove plate 34 has a groove therein which receives a sprung detent member 52 of the slider 32, the function of which will be described later. Also provided on the slider 32 is a ratchet driver 44 which engages with a ratchet wheel 40, the operation of which will be described later. A ratchet driver guide 38 is provided on the housing base 42. The back housing member 46 (and front housing member 64 - Figure 6) have cartridge tracks therein, along which the cartridge 12 can move. In the start position shown in Figure 5a, the user presses the release button (not shown) where indicated by the arrow 30. This releases a catch 58 (Figure 6) on the slider 32. A spring 60 (Figure 6) pushes the plunger 6 and the slider 32 in the direction of the large arrow in Figure 5b. The slider 32 has a hockey stick-shaped slot 31 in which is received the pusher 16. As the slider 32 travels, it moves the pusher 16 in the direction of the small arrow 50 shown in Figure 5b. At 6 mm before the end of travel, the pusher 16 is removed from the cartridge 12. In the final 6 mm of travel the ratchet driver 44 indexes the ratchet wheel by one position, and advances the cartridge 12 so that the foil 20 on the first magazine 10 is slit at one end. The user then pushes the slider 32 to an intermediate position (Figure 5c) and in so doing, causes the pusher 16 to move back, with its cutting surfaces cutting the foil as previously described, and pushing the uppermost test strip out of the housing 2. The slider 32 is held in the intermediate position by engagement of the sprung detent member 52 in the groove of the groove plate 34. The detent member 52 and groove plate 34 operate in the manner of a ballpoint pen advancing and retracting mechanism, with the detent member 52 cycling around the groove in the course of one test cycle. After a reading has been taken, the plunger 6 is then fully pushed in by the user (Figure 5d), causing the slider to return to the position shown in Figure 5a and causing the pusher to eject the used test strip 8. The slider 32 is now held in place until the next test reading is required. Figure 6 shows some of the components more clearly, including the chassis cartridge end 70 which has a cartridge drive spring 68. A pusher "parking slot"plug 56 is mounted in the back housing member 46, where the pusher 16 is kept away from the cartridge 12 to permit movement of the cartridge. An optional sensor exit slot plug 66 is provided in the front housing member 64. A PCB 54 provides the meter electronics. When the test device is first used, and when a magazine is first opened up, a microprocessor on the PCB starts a timer counting down whereby the display 4 indicates when a particular magazine has exceeded its recommended life, or in the event that all magazines have exceeded their shelf life.

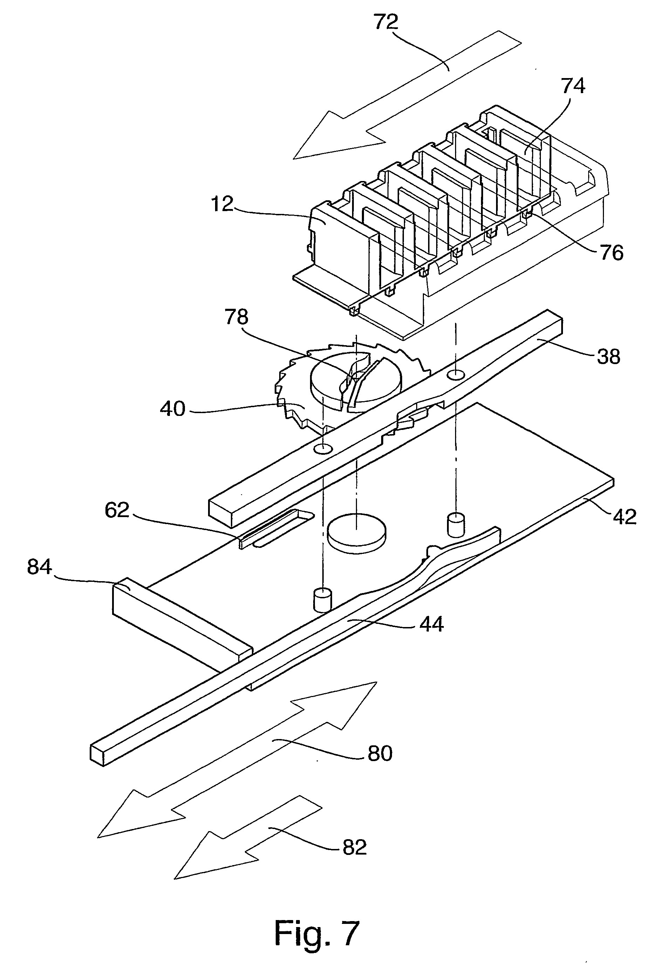

Figure 7 shows the cartridge advance mechanism as an exploded schematic, with the cartridge in half-section. The cartridge 12 is urged by the spring 68 in the direction of the arrow 72. Underneath the cartridge 12 there is provided a plurality of cartridge location pegs 76, which are spaced apart by the width of the magazines, ie the distance between the centre lines of the magazines 10. There is a peg 76 for each cavity 74, and an additional leading peg on a front lip of the cartridge 12. The ratchet wheel 40 has a keyway 78 thereon for the cartridge location pegs 76 to pass through as the cartridge 12 advances. The ratchet wheel is driven by the ratchet driver 44 which, cooperating with a pawl 62, drives the ratchet wheel 40 in a clockwise direction as viewed in Figure 7. The ratchet driver 44 reciprocates in both directions (80), but is sprung only in the direction indicated by the arrow 82. A cartridge final stop member 84 is provided at the proximal end of the housing base 42.

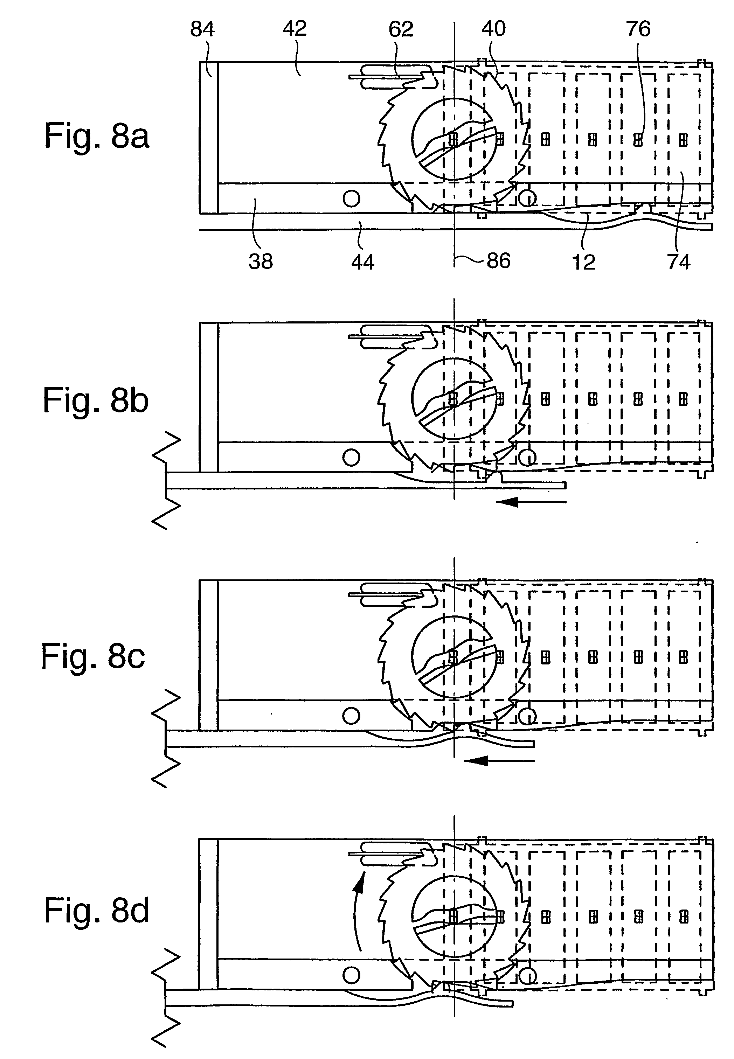

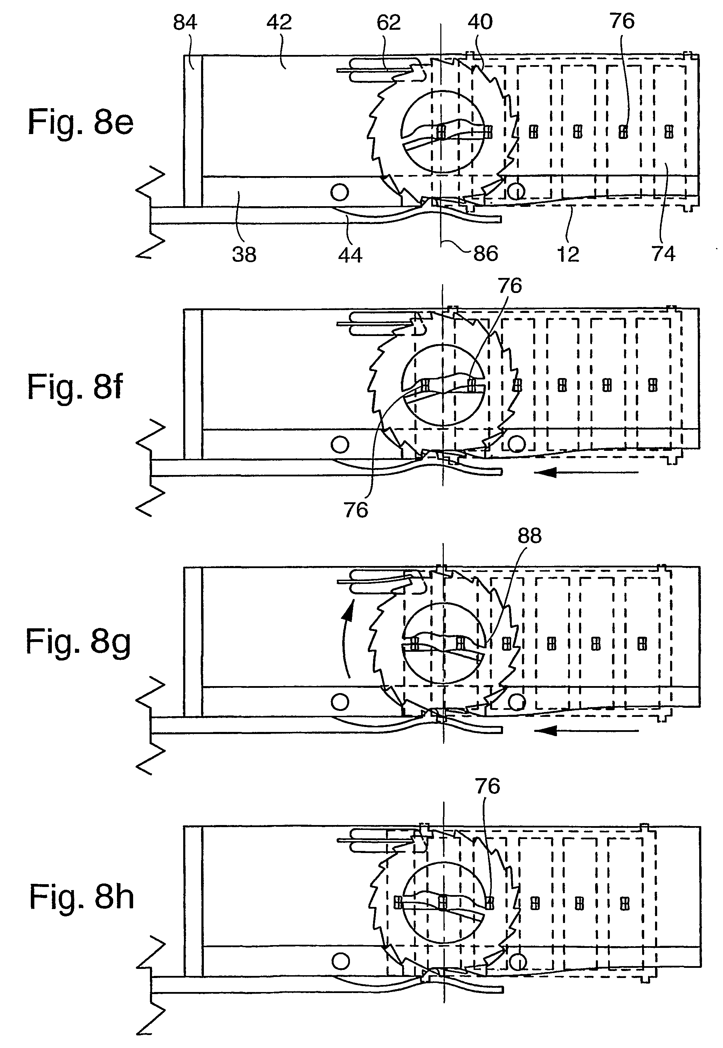

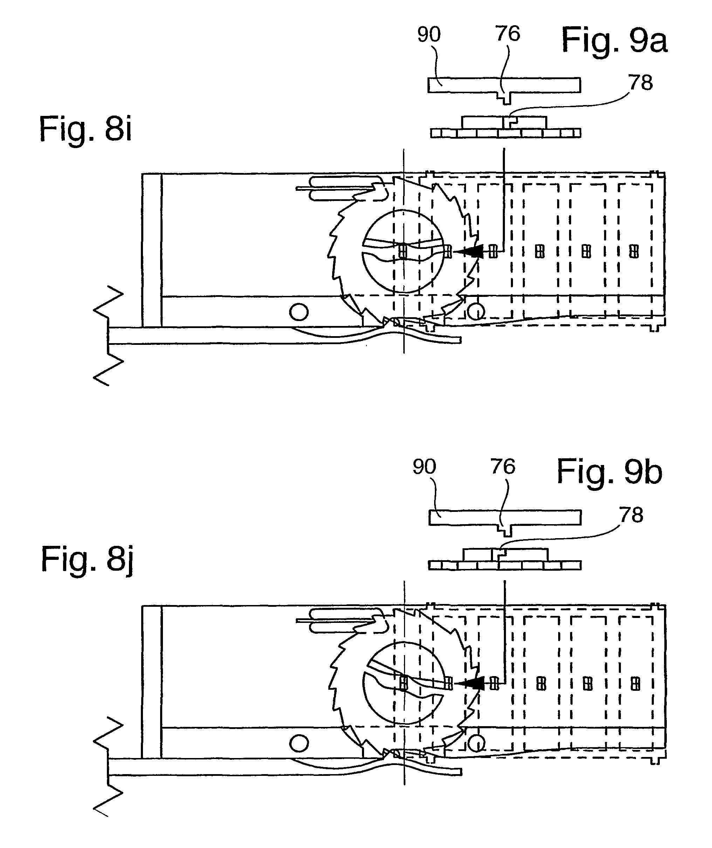

After each advance of the cartridge 12, it remains stationary until the test strips in a magazine have been used up. At this point the cartridge 12 advances by the width of a magazine. The mechanism for controlling advance of the cartridge 12 is described with reference to Figures 8 and 9. Starting from the position shown in Figure 8a, the ratchet mechanism is in a rest position ready for use. The centre line 86 of the active magazine 12 is in the keyway of the ratchet wheel 40. The user presses the release button and the ratchet driver 44 travels in the sprung direction (arrowed - Figure 8b). The ratchet driver 44 engages the ratchet wheel (Figure 8c) and rotates or indexes the ratchet wheel by one place (arrowed - Figure 8d). Figures 8d through 8h shows the cartridge advancement sequence. As the ratchet wheel 40 advances from the position shown in Figure 8e to that of Figure 8f, the two foremost location pegs 76 of cartridge 12 are freed to advance through the keyway 78. The location peg 76 furthest to the left in Figure 8f acts against the inclined surface of the keyway 78 while exiting the keyway. This forces the ratchet wheel 40 to rotate one place (Figure 8g) while the second location peg 76 is moved to the centre of the ratchet wheel. The rotation of the ratchet wheel 40 presents a solid stop wall at the point 88 to the oncoming location peg. At the position shown in Figure 8h, the cartridge has advanced by one magazine width, and the location peg 76 of the magazine behind the new active magazine comes to rest against the ratchet wheel stop wall, aligning the next magazine ready for use.

When the cartridge 12 is loaded in the housing during manufacture, the front location peg 76 is located in the keyway 78 and the ratchet wheel 40 is located so that the first time the ratchet wheel is indexed, the foremost magazine 10 is advanced as describe above, and the seal is cut by the blade 22.

Because there are 20 test strips per magazine, and 20 teeth on the ratchet wheel, the ratchet wheel is constructed to permit cartridge advance only once during a complete (360°) rotation. Figures 8i and 9a show how, with the ratchet wheel at position 10 (180° rotation), the cartridge 12 is prevented from premature advancement. The cartridge location peg 76 is prevented from entering the ratchet wheel keyway 78 through incorrect orientation and misalignment. The cartridge base 90 and location peg 76 are shown separated from the ratchet wheel in Figures 9a and 9b for clarity. With the ratchet wheel at position 11 (Figures 8j and 9b) the location peg 76 is also misaligned with the ratchet keyway 78 and the cartridge does not advance.

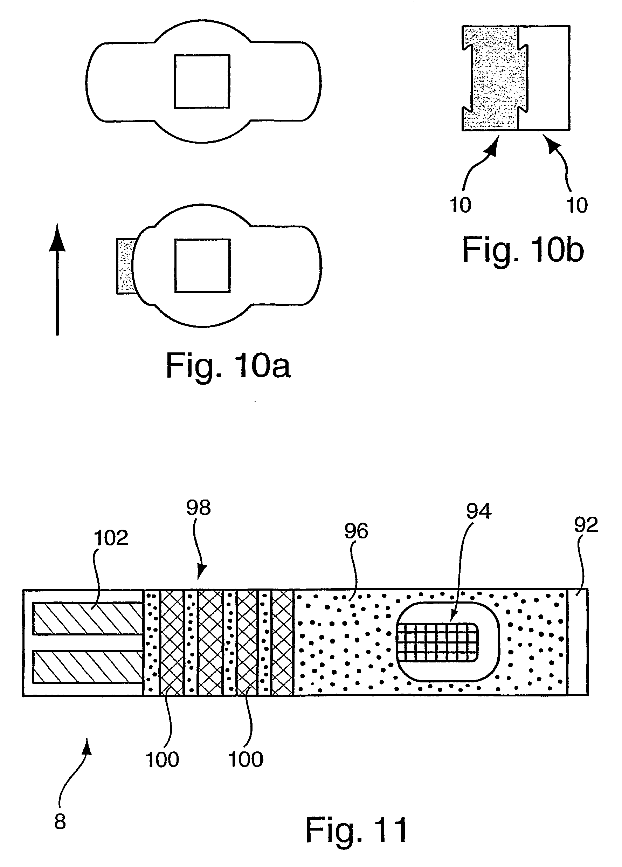

An alternative embodiment is illustrated with respect to Figure 10. Here, the magazines are releasably connected together, in this example by a dovetail joint (Figure 10b). Used magazines 10 project through an opening in the housing and can be removed by the user. Figure 10a shows plan views before (top) and after (bottom) a used magazine has been slid off from its neighbour. In this embodiment the housing can be made smaller because it need not accommodate used magazines.

The test strip 8 shown in Figure 11 comprises a planar base member 92, in this example of poly(butylene terephthalate) (PBT) (Valox® FR-1 from GE Plastics). The strip is 30 mm x 5.5 mm, and 0.5 mm thick. A working area 94 is of conventional construction, comprising a plurality of electrodes, a reagent layer in intimate contact with the electrodes, and a mesh layer for spreading out a drop of fluid to be received on the working area. Electrode tracks 102, for example of carbon, in the non-working area 98 of the test strip are connected to the electrodes in the working area 94 in known manner. Also in known manner, a dielectric layer 96 is printed around the working area 94 so as to overlie a portion of the electrode tracks 102, leaving just the ends of the tracks exposed for connection to corresponding 26 electrodes on the meter. The layers are applied to the base member as inks, by screen printing. Each ink layer is about 10 to 20 µm thick, and the mesh is about 59 to 67 µm thick. The working area 94 has a total thickness which is about 100 µm thicker than the non-working area 98 up to the dielectric layer 96.

To increase the thickness of parts of the non-working area, a high relief ink 100 has been printed in four strips. The high relief ink has a dried thickness such that the total thickness of the non-working area to which the high relief ink 100 has been applied is slightly greater than the total thickness of the test strip in the working area 94. Thus, when a stack of such test strips 8 is formed, and a compressive load is applied to the stack by the spring 14, the working area 94 will not bear all the compressive load. Scuffing of the test area will be reduced compared to a conventional test strip in which the working area stands proud of the non-working area.

Although this embodiment has been illustrated with reference to the use of a high relief ink printed in strips, it will be understood that it is not limited to this embodiment. The ink could be printed as a continuous block, and it could entirely surround the working area if desired. Instead of, or in addition to, the high relief ink, other means could also be provided to increase the thickness of the non-working area, for example: an applied pad or tape; embossing of the base layer or an intermediate layer; or an extension of the mesh layer from the working area into the non-working area.

Although the invention has been described with reference to a test device for measuring blood glucose concentration, it is to be understood that the invention is not limited to this application. The invention may be used in the determination of any analyte in a fluid by the use of suitable reagents in the test strip. Such reagents are well known to those skilled in the art.

## Claims

1. A test device for testing of analyte concentration in a fluid to be applied thereto, the device comprising:
a) a plurality of test members (8) arranged in at least one stack, each of said test members (8) carrying reagent means for producing an electrical signal in response to the concentration of analyte in an applied fluid, each of said test members (8) having a plurality of electrode tracks (102) for transmitting said electrical signal;
b) a housing (2) having electrodes (26) disposed therein for engaging with the electrode tracks on a test member (8) at an engagement location;
c) a meter connected to the said electrodes (26) and disposed at least partly in the housing (2), having electronics means (4) for producing a signal output which is dependent on the electrical signal from a test member (8) when the test member (8) is engaged with the said electrodes (26);
d) a pusher (16) which is adapted to push a single test member (8) from the stack and into the engagement location where it can engage with the said electrodes (26) and where the test member (8) can be accessed to apply a fluid thereto;
e) an actuation member (6) operably connected to the pusher (16), the said actuation member (6) being operable by a user to move the pusher (16);
f) the or each stack of test members (8) being enclosed in a magazine (10) which is initially sealed by a moisture impermeable seal (20); **characterised by**:
g) blade means (22) mounted in the housing (2) for slitting the said seal (20); and
h) at least one cutting surface (24) on the pusher (16) for cutting the seal (20);
i) wherein movement of the or each magazine (10) from an initial position where the seal (20) is intact to a position where the pusher (16) can push a test member (8) from the stack therein will cause the blade means (22) to cut the seal (20) to form a slit through which a test member (8) can pass when pushed by the pusher (16).

2. A device as claimed in claim 1, wherein the test members (8) are arranged in a plurality of stacks, each one enclosed in a magazine (10) which is initially sealed by a moisture impermeable seal (20), the magazines (10) being movable so as to enable the blade means (22) to slit the seal (20) of each in turn as the magazine (10) reaches a position in which the pusher (16) can push a test member (8) therefrom.

3. A device as claimed in claim 1 or claim 2, wherein operation of the actuation member (6) causes the pusher (16) to engage with the electrodes (26) and bring them into contact with the electrode tracks (102) on the test member (8) when the test member (8) is in the engagement location.

4. A device as claimed in any one of the preceding claims, wherein further operation of the actuation member (6) causes the pusher (16) to push a test member (8) from the engagement location and eject the test member (8) from the housing (2).

5. A device as claimed in claim 2, wherein the or each magazine (10) is housed in a cartridge (12).

6. A device as claimed in claim 5, wherein the cartridge (12) is urged by spring means (68) towards a ratchet wheel (40) which has a keyway (78) therein, the cartridge (12) being provided with a plurality of spaced-apart location pegs (76) for locating in the keyway (78) and the ratchet wheel (40) only permitting entry of a location peg (76) when the ratchet wheel (40) is in a defined orientation.

7. A device as claimed in claim 6, wherein operation of the actuating member (6) causes indexing of the ratchet wheel (40).

8. A device as claimed in any one of the preceding claims, further comprising a sliding member (32) which has an angled slot (31) therein which provides a cam surface that bears against the pusher (16), whereby movement of the sliding member (32) in a first direction causes movement of the pusher (16) in a second direction.

9. A device as claimed in any one of the preceding claims, further comprising spring means (14) which urge the stack of test members towards the seal (20).

10. A device as claimed in any one of the preceding claims, further comprising means (34, 52) for releasably detaining the pusher (16) when the test member (8) is in the engagement location.

11. A device as claimed in any one of the preceding claims, further comprising a processor and means for initiating a timer at a predetermined time or when a magazine is first opened, the processor being programmed to provide a visible warning or message if a magazine (10) which is in use exceeds its shelf life or if unopened magazines (10) exceed their shelf life.

12. A test device as claimed in any one of the preceding claims, wherein the analyte to be tested for is glucose and the fluid to be applied is blood.

13. A test device as claimed in claim 2, wherein the magazines (10) are releasably connected together and wherein the housing (2) has an opening through which used magazines (10) will project.

14. A test device as claimed in any one of the preceding claims, wherein each test member (8) in the or each stack comprises a base member (92) having a working area (94) to which the fluid is to be applied, containing the reagent means, and a non-working area (98) adjacent to the working area (94), wherein the total thickness of the test member (8) in at least a portion of the non-working area (98) is at least as great as the total thickness of the test member (8) in the working area (94).

15. A test device as claimed in claim 14, wherein the total thickness of the test member (8) in at least a part of the non-working area (98) is greater than the total thickness of the test member (8) in the working area (94).

## Patentansprüche

1. Testvorrichtung zur Bestimmung der Analytkonzentration in einem darauf aufgetragenen Fluid, wobei die Vorrichtung aufweist:
a) eine Vielzahl von Testgliedern (8), die in mindestens einem Stapel angeordnet sind, wobei jedes dieser Testglieder (8) Reagenzmittel zur Herstellung eines elektrischen Signals In Ansprechen auf die Analytkonzentration in einem aufgetragenen Fluid trägt und jedes dieser Testglieder (8) über eine Vielzahl von Elektrodenbahnen (102) zur Übermittlung dieses elektrischen Signals verfügt,
b) ein Gehäuse (2) mit darin angeordneten Elektroden (26) zum in Eingriffkommen mit den Elektrodenbahnen auf einem Testglied (8) an einer Eingriffsposition,
c) einen Zähler, der mit diesen Elektroden (26) verbunden und mindestens teilweise in dem Gehäuse (2) angeordnet ist sowie über elektronische Mittel (4) zur Herstellung eines Signaloutputs in Abhängigkeit von dem elektrischen Signal von einem Testglied (8), wenn das Testglied (8) mit den Elektroden (26) im Eingriff ist, verfügt,
d) einen Ausstoßer (16), der ein einzelnes Testglied (8) aus einem Stapel zu der Eingriffsposition schieben kann, in der es mit den Elektroden (26) in Eingriff kommen kann und in der das Testglied (8) zum Auftragen eines Fluids darauf zugänglich ist,
e) ein Betätigungsglied (6), das mit dem Ausstoßer (16) in Wirkverbindung steht, wobei das Betätigungsglied (6) von einem Benutzer zum Bewegen des Ausstoßers (16) betätigt werden kann,
f) der oder jeder Stapel von Testgliedern (8) in einem Magazin (10) eingeschlossen ist, das ursprünglich durch ein feuchtigkeitsimpermeables Siegel (20) versiegelt ist,
**gekennzeichnet durch**,
g) Schneidemittel (22), die in dem Gehäuse (2) zum Schlitzen des Siegels (20) befestigt sind und
h) mindestens eine Schneidfläche (24) auf dem Ausstoßer (16) zum Schneiden des Siegels (20),
i) wobei die Bewegung des oder jedes Magazins (10) von einer anfänglichen Position, in der das Siegel (20) unversehrt ist, zu einer Position, in der der Ausstoßer (16) ein Testglied (8) aus dem Stapel stoßen kann, das Schneidemittel (22) dazu bringt, das Siegel (20) unter Bildung eines Schlitzes zu schneiden, **durch** den ein Testglied (8) passieren kann, wenn es von dem Ausstoßer (16) gestoßen wird.

2. Vorrichtung nach Anspruch 1,
bei der die Testglieder (8) in einer Vielzahl von Stapeln angeordnet sind, jeden in einem Magazin (10) eingeschlossen ist, das anfänglich durch ein feuchtigkeitsimpermeables Siegel (20) versiegelt ist, die Magazine (10) derart bewegt werden können, dass sie das Schneidemittel (22) in die Lage versetzen, das Siegel (20) von jedem zu schlitzen, wenn das Magazin (10) eine Position erreicht, in der der Ausstoß (16) ein Testglied (8) daraus ausstoßen kann.

3. Vorrichtung nach Anspruch 1 oder 2,
bei der die Betätigung des Betätigungsgliedes (6) den Ausstoßer (16) dazu veranlasst, mit den Elektroden (26) in Eingriff zu kommen und sie in Kontakt mit den Elektrodenbahnen (102) auf dem Testglied (8) zu bringen, wenn das Testglied (8) in der Eingriffsposition ist.

4. Vorrichtung nach einem der vorhergehenden Ansprüche,
bei der die weitere Betätigung des Betätigungsgliedes (6) den Ausstoßer (16) dazu veranlasst, ein Testglied (8) aus der Eingriffsposition zu schieben und das Testglied (8) aus dem Gehäuse (2) herauszuschieben.

5. Vorrichtung nach Anspruch 2,
bei der das oder jedes Magazin (10) in einer Kartusche (12) untergebracht ist.

6. Vorrichtung nach Anspruch 5,
bei der die Kartusche (12) durch Federmittel (68) in Richtung eines Klinkenrades (40) bewegt wird, das eine darin angebrachte Keilnut (78) besitzt, die Kartusche (12) mit einer Vielzahl von voneinander beabstandeten Einrastzapfen (76) zum Einrasten in der Keilnut (78) ausgestattet ist und das Klinkenrad (40) nur dann den Eintritt des Einrastzapfens (76) erlaubt, wenn sich das Klinkenrad (40) in einer definierten Orientierung befindet.

7. Vorrichtung nach Anspruch 6,
bei der die Betätigung des Betätigungsgliedes (6) eine Schaltung des Klinkenrades (40) bewirkt.

8. Vorrichtung nach einem der vorhergehenden Ansprüche,
die außerdem ein Gleitelement (32) aufweist, das einen gewinkelten Schlitz (31) darin aufweist, der eine Mitnehmerfläche besitzt, welche gegen den Ausstoßer (16) anliegt, wobei eine Bewegung des Gleitelementes (32) in eine erste Richtung eine Bewegung des Ausstoßers (16) in eine zweite Richtung hervorruft.

9. Vorrichtung nach einem der vorhergehenden Ansprüche,
die außerdem Federmittel (14) aufweist, welche den Stapel von Testgliedern in Richtung des Siegels (20) zwingen.

10. Vorrichtung nach einem der vorhergehenden Ansprüche,
die außerdem Mittel (34, 52) aufweist, welche den Ausstoßer (16) lösbar zurückhalten, wenn sich das Testglied (18) in der Eingriffsposition befindet.

11. Vorrichtung nach einem der vorhergehenden Ansprüche,
die außerdem einen Prozessor und Mittel zum Starten eines Zeitmessers zu einem vorher bestimmten Zeitpunkt oder sobald ein Magazin zum ersten Mal geöffnet wird, aufweist, wobei der Prozessor derart programmiert ist, dass er eine sichtbare Warnung oder Nachricht bereitstellt, falls ein in Benutzung befindliches Magazin (10) seine Haltbarkeitszeit überschritten hat oder falls nicht geöffnete Magazine (10) ihre Haltbarkeitszeit überschritten haben.

12. Testvorrichtung nach einem der vorhergehenden Ansprüche,
bei der der zu testende Analyt Glukose ist und das aufzutragende Fluid Blut ist.

13. Testvorrichtung nach Anspruch 2,
bei der die Magazine (10) lösbar miteinander verbunden sind und bei der das Gehäuse (2) eine Öffnung besitzt, aus der verbrauchte Magazine (10) ausgestoßen werden.

14. Testvorrichtung nach einem der vorhergehenden Ansprüche,
bei der jedes Testglied (8) in dem oder jedem Stapel ein Basisglied (32) mit einer Arbeitszone (94), auf das das Fluid aufgetragen wird, welches die Reagenzmittel enthält, und eine Nicht-Arbeitszone (98) benachbart zu der Arbeitszone (94) aufweist und In der die Gesamtdicke der Testglieder (8) in mindestens einem Abschnitt der Nicht-Arbeitszone (98) mindestens so groß ist wie die Gesamtdicke des Testglieds (8) in der Arbeitszone (94).

15. Testvorrichtung nach Anspruch 14,
bei der die Gesamtdicke des Testglieds (8) in mindestens einem Teil der Nicht-Arbeitszone (98) größer ist als die Gesamtdicke des Testglieds (8) in der Arbeitszone (94).

## Revendications

1. Dispositif d'essai pour tester la concentration en substance à analyser dans un fluide à appliquer à celui-ci, le dispositif comprenant :
a) une pluralité d'éléments de test (8) agencés dans au moins une pile, chacun desdits éléments de test (8) portant des moyens réactifs pour produire un signal électrique en réponse à la concentration en substance à analyser dans un fluide appliqué, chacun desdits éléments de test (8) ayant une pluralité de pistes d'électrodes (102) pour transmettre ledit signal électrique ;
b) un boîtier (2) ayant des électrodes (26) disposées à l'intérieur pour venir en prise avec les pistes d'électrodes sur un élément de test (8) à un emplacement de mise en prise ;
c) un compteur connecté auxdites électrodes (26) et disposées au moins en partie dans le boîtier (2), ayant des moyens électroniques (4) pour produite une sortie de signal qui est dépendante du signal électrique provenant d'un élément de test (8) lorsque l'élément de test (8) vient en prise avec lesdites électrodes (26) ;
d) un poussoir (16) qui est adapté pour pousser un élément de test (8) unique depuis la pile et dans l'emplacement de mise en prise où il peut venir en prise avec lesdites électrodes (26) et dans lequel l'élément de test (8) peut être atteint pour appliquer un fluide sur celui-ci ;
e) un élément d'actionnement (6) connecté de façon opérationnelle au poussoir (16), ledit élément d'actionnement (6) étant actionnable par un utilisateur pour déplacer le poussoir (16) ;
f) la ou chaque pile d'éléments de test (8) étant incluse dans un magasin (10) qui est initialement scellé par un joint (20) imperméable à l'humidité ; **caractérisé par** :
g) des moyens formant lames (22) montés dans le boîtier (2) pour fendre ledit joint (20) ; et
h) au moins une surface de coupe (24) sur le poussoir (16) pour couper le joint (20) ;
i) dans lequel le mouvement du ou de chaque magasin (10) depuis une position initiale dans laquelle le joint (20) est intact vers une position dans laquelle le poussoir (16) peut pousser un élément de test (8) depuis la pile dans celui-ci, amène les moyens formant lames (22) à couper le joint (20) pour former une fente à travers laquelle un élément de test (8) peut passer en étant poussé par le poussoir (16).

2. Dispositif selon la revendication 1, dans lequel les éléments de test (8) sont agencés dans une pluralité de piles, chacune enfermée dans un magasin (10) qui est initialement scellé par un joint (20) imperméable à l'humidité, les magasins (10) étant déplaçables afin de permettre aux moyens formant lames (22) de fendre le joint (20) de chacun l'un après l'autre alors que le magasin (10) atteint une position dans laquelle le poussoir (16) peut pousser un élément de test (8) depuis celui-ci.

3. Dispositif selon la revendication 1 ou 2, dans lequel le fonctionnement de l'élément d'actionnement (6) amène le poussoir (16) à venir en prise avec les électrodes (26) et les amène en contact avec les pistes d'électrodes (102) sur l'élément de test (8) lorsque l'élément de test (8) est dans l'emplacement de mise en prise.

4. Dispositif selon l'une quelconque des revendications précédentes, dans lequel un autre fonctionnement de l'élément d'actionnement (6) amène le poussoir (16) à pousser un élément de test (8) depuis l'emplacement de mise en prise et à éjecter l'élément de test (8) depuis le boîtier (2).

5. Dispositif selon la revendication 2, dans lequel le ou chaque magasin (10) est placé dans une cartouche (12).

6. Dispositif selon la revendication 5, dans lequel la cartouche (12) est poussée par des moyens formant ressorts (68) vers une roue à rochet (40) qui a une rainure de clavette (78) à l'intérieur, la cartouche (12) est pourvue d'une pluralité de clavettes d'emplacement (76) espacés à des fins de mise en place dans la rainure de clavette (78) et la roue à rochet (40) autorisant uniquement l'entrée d'une clavette d'emplacement (76) lorsque la roue à rochet (40) est dans une orientation définie.

7. Dispositif selon la revendication 6, dans lequel le fonctionnement de l'élément d'actionnement (6) entraîne l'indexation de la roue à rochet (40).

8. Dispositif selon l'une quelconque des revendications précédentes, comprenant en outre un élément coulissant (32) qui a une fente angulaire (31) dans celui-ci qui fournit une surface de came qui repose contre le poussoir (16), moyennant quoi le mouvement de l'élément coulissant (32) dans une première direction entraîne le mouvement du poussoir (16) dans une seconde direction.

9. Dispositif selon l'une quelconque des revendications précédentes, comprenant en outre des moyens formant ressorts (14) qui poussent la pile des éléments de test (8) vers le joint (20).

10. Dispositif selon l'une quelconque des revendications précédentes, comprenant en outre des moyens (34, 52) pour retenir de façon détachable le poussoir (16) lorsque l'élément de test (8) est dans l'emplacement de mise en prise.

11. Dispositif selon l'une quelconque des revendications précédentes, comprenant en outre un processeur et des moyens pour initier un temporisateur à un moment prédéterminé ou lorsqu'un magasin est ouvert en premier, le processeur étant programmé pour émettre un avertissement ou message visible si un magasin (10) qui est utilisé dépasse sa durée de stockage ou si des magasins (10) non ouverts dépassent leur durée de stockage.

12. Dispositif d'essai selon l'une quelconque des revendications précédentes, dans lequel la substance à analyser à tester est du glucose et le fluide à appliquer est du sang.

13. Dispositif d'essai selon la revendication 2, dans lequel les magasins sont connectés de façon détachable ensemble et dans lequel le boîtier (2) a une ouverture à travers laquelle les magasins (10) utilisés font saillie.

14. Dispositif d'essai selon l'une quelconque des revendications précédentes, dans lequel chaque élément de test (8) dans la ou chaque pile comprend un élément de base (92) ayant une zone de travail (94) à laquelle le fluide est appliqué, contenant les moyens réactifs, et une zone de non-travail (98) adjacente à la zone de travail (94), dans lequel l'épaisseur totale de l'élément de test (8) dans au moins une partie de la zone de non-travail (98) est au moins aussi importante que l'épaisseur totale de l'élément de test (8) dans la zone de travail (94).

15. Dispositif d'essai selon la revendication 14, dans lequel l'épaisseur totale de l'élément de test (8) dans au moins une partie de la zone de non-travail (98) est supérieure à l'épaisseur totale de l'élément de test (8) dans la zone de travail (94).
